# EUROPEAN PATENT APPLICATION

(11) **EP 2 123 776 A1**
(43) Date of publication of application: **25.11.2009**
(21) Application number: 08290477.2
(22) Date of filing: 23.05.2008
(51) Int. Cl.: C12Q 1/68

(54) **Process for detecting botulinum neurotoxin producing organisms**

(71) Applicant: Agence Française de Securité Sanitaire des Aliments, 94700 Maisons-Alfort Cedex (FR)
(72) Inventor: Fach, Patrick, 94000 Creteil (FR); Micheau, Pierre, 63118 Cebazat (FR); Perelle, Sylvie, 94220 Charenton-le-Pont (FR)
(74) Representative: Thomas, Dean

(57) **Abstract**

The present invention relates to methods and processes to detect organisms which produce botulinum neurotoxins. In particular the invention relates to a process for the detection of neurotoxin producing clostridia in a sample, wherein this process comprises contacting the sample or DNA isolated therefrom with a pair of primers derived from the NTNH gene and detecting the presence or the absence of an amplification product.

## Description

The present invention relates to methods to detect organisms which produce botulinum neurotoxins. In particular the present invention relates to a set of assays which simultaneously detect in a sample, genes involved in the biosynthetic pathway of botulinum neurotoxins A, B, E, F and thereby indirectly detect the presence of botulinum toxin producing organisms such as *Clostridium botulinum*, *Clostridium baratii* and *Clostridium butyricum.*

Botulinum neurotoxins (BoNT) are produced by phenotypically and genetically different Clostridium species including *Clostridium botulinum* and some strains of *Clostridium baratii* (Mc Croskey et al., 1991) and *Clostridium butyricum* (Mc Croskey et al., 1986). BoNTs are the most potent biological and chemical substances known and are responsible for botulism, which is characterized by severe flaccid paralysis.

BoNTs are divided into seven toxin types (A to G) according to their antigenic properties. Toxin types A, B, E and more rarely F cause human botulism, whereas toxin types C and D are mainly responsible for animal botulism (Popoff, 1995, Herreros et al. 1999; Schiavo et al. 2000). Although less common, bivalent strains that express two different BoNT types exist and are designated by the predominant toxin produced (Ab, Ba, Af, Bf) (Gimenez & Gimenez 1993). Other bivalent strain variants such as the A1(B) strains contain both BoNT/A and BoNT/B genes but express only BoNT/A (Rodriguez et al. 1998).

Sequencing of the BoNT genes from multiple strains of serotypes A, B, E and F showed significant sequence variation within each serotype. While serotypes differ by about 35-70%, subtype differences range from approximately 2-32%. Four distinct lineages within each of the BoNT A and B serotypes and five distinct lineages of serotype E strains have been identified (Hill et al. 2007). The nucleotide sequences of the seven toxin genes of the serotypes have been compared and showed various degrees of interrelatedness and recombination.

The non-toxic nonhemagglutinin (NTNH) protein that is closely associated with the BoNT protein is present in all toxin clusters but its function is not fully understood. It is thought that this protein assists in stability of the neurotoxin within the acidic and protease-rich environment of the stomach and assists in transport of the toxin from the intestinal area to the bloodstream (Maksymowych et al. 1999).

At the present time, the identification of *C*. *botulinum* (and other BoNT producing clostridia organisms) requires the production of toxins in culture and testing by mouse bioassay with neutralization of the toxin with type-specific antitoxin. This process is time consuming and labour intensive, and thus there is a need to develop more rapid nucleic acid based assays to presumptively identify BoNT producing clostridia organisms.

The nucleotide diversity (identity ranges from 56-76%) among the BoNT genes (Hauser et al. 1995) present a significant challenge in designing universal primers and/or probes that enable detection of these genes by real-time PCR (Fach et al. 2002). Nonetheless, BoNTs are generated as part of a progenitor toxin complex and a conserved component among serotypes is the non-toxic nonhemagglutinin (NTNH). East and Collins (1994) demonstrated that the gene encoding NTNH is present in all strains that produce BoNTs and absent from strains that are non-toxic.

Nucleotide sequence analysis of the cluster of genes associated with the BoNT gene demonstrates the presence of the NTNH gene directly upstream of the BoNT gene in all toxin types tested. Moreover, the BoNT and NTNH genes are likely co-transcribed as demonstrated by the identification of a transcript with a size that is approximately the sum of both genes (Henderson et al. 1996). Raphael & Andreadis (2007) report a high level of similarity (amino acid identity ranges from 70-99%) of the NTNH amino acid sequence from toxic strains of *C*. *botulinum* and describe a PCR assay for screening BoNT producing strains by detecting the NTNH gene. This assay uses a single labelled probe targeting a highly conserved region of the NTNH gene; however, the regions flanking the probe site containing a too high level of nucleotide diversity for allowing the design of consensus primers, it is necessary to use a combination of seven different primers.

The inventors have now identified another highly conserved region of the NTNH gene and have developed a new PCR assays targeting this region and allowing the simultaneous detection of *C*. *botulinum* A, B, E or F (and other BoNT producing clostridia). In contrast with the PCR assay developed by Raphael & Andreadis, the assay developed by the inventors to detect the NTNH gene uses a single pair of consensus primers.

Therefore the present invention relates to a process for the detection of neurotoxin producing clostridia in a sample, wherein said process comprises contacting said sample or DNA isolated therefrom with a pair of primers comprising:
- a primer defined by the sequence SEQ ID NO: 16, or a fragment of at least 15 nucleotides thereof;
- a primer defined by the sequence SEQ ID NO: 17, or a fragment of at least 15 nucleotides thereof;
and detecting the presence or the absence of an amplification product.

The presence of said amplification product is indicative of the presence of neurotoxin producing clostridia. Typically, said amplification product has a length of 80 bp.

Preferably said amplification product is detected using a probe defined by the sequence SEQ ID NO: 18 or a fragment of at least 15 nucleotides thereof.

A second PCR assay developed by the inventors targets highly conserved regions between the BoNT genes A, B, E, and F. Using this PCR assay, only one set of primers is required to detect the presence of any of the of BoNT genes A, B, E, F in a sample.

This second PCR assay involves the use of a pair of primers comprising:
- a primer defined by the sequence SEQ ID NO: 13, or a fragment of at least 15 nucleotides thereof;
- a primer defined by the sequence SEQ ID NO: 14, or a fragment of at least 15 nucleotides thereof.

If any of the BoNT genes A, B, E, or F is present in the sample tested, these primers give an amplification product having a length of 188 to 200 bp, which is indicative of the presence of neurotoxin producing clostridia.

Preferably said amplification product is detected using a probe defined by the sequence SEQ ID NO: 15 or a fragment of at least 15 nucleotides thereof.

Advantageously, the second PCR assay is used in combination with the first PCR assay referred above.

For tracing BoNT/A to BoNT/F producing clostridia, the inventors have further developed four PCR assays, each being specific to one of the botulinum neurotoxin genes A, B, E, or F, and enabling a toxin type-specific identification.

A PCR assay for detecting specifically BoNT/A involves the use of a pair of primers comprising:
- a primer defined by the sequence SEQ ID NO: 1, or a fragment of at least 15 nucleotides thereof;
- a primer defined by the sequence SEQ ID NO: 2, or a fragment of at least 15 nucleotides thereof.

If the gene BoNT/A is present in the sample tested, these primers give an amplification product having a length of 75bp. Advantageously, said amplification product is detected using a probe defined by the sequence SEQ ID NO: 3 or a fragment of at least 15 nucleotides thereof.

A PCR assay for detecting specifically BoNT/B involves the use of a pair of primers comprising:
- a primer defined by the sequence SEQ ID NO: 4, or a fragment of at least 15 nucleotides thereof;
- a primer defined by the sequence SEQ ID NO: 5, or a fragment of at least 15 nucleotides thereof.

If the gene BoNT/B is present in the sample tested, these primers give an amplification product having a length of 172 bp. Advantageously, said amplification product is detected using a probe defined by the sequence SEQ ID NO: 6 or a fragment of at least 15 nucleotides thereof.

A PCR assay for detecting specifically BoNT/E involves the use of a pair of primers comprising:
- a primer defined by the sequence SEQ ID NO: 7, or a fragment of at least 15 nucleotides thereof;
- a primer defined by the sequence SEQ ID NO: 8, or a fragment of at least 15 nucleotides thereof.

If the gene BoNT/E is present in the sample tested, these primers give an amplification product having a length of 115 bp. Advantageously, said amplification product is detected using a probe defined by the sequence SEQ ID NO: 9 or a fragment of at least 15 nucleotides thereof.

A PCR assay for detecting specifically BoNT/F involves the use of a pair of primers comprising:
- a primer defined by the sequence SEQ ID NO: 10, or a fragment of at least 15 nucleotides thereof;
- a primer defined by the sequence SEQ ID NO: 11, or a fragment of at least 15 nucleotides thereof.

If the gene BoNT/F is present in the sample tested, these primers give an amplification product having a length of 112 bp. Advantageously, said amplification product is detected using a probe defined by the sequence SEQ ID NO: 12 or a fragment of at least 15 nucleotides thereof.

According to a particularly preferred embodiment of the invention, at least one of these PCR assays for detecting specifically BoNT/A, BoNT/B, BoNT/C, or BoNT/F is used in combination with the first and/or the second PCR assay referred above.

The invention also encompasses the primer pairs and the probes defined above, as well as kits associating these primer pairs and these probes, eventually associated with reagents to perform an amplification reaction, and optionally for detecting the products thereof. These kits may also comprise also instructions for performing said amplification reaction. The amplification products obtained from the NTNH gene or from the BoNT gene, using the primers of the invention, are also part of the invention.

Some of these primers and probes can be degenerate oligonucleotides, i.e. mixtures of similar oligonucleotides representing all the possible variants of a consensus sequence. One can also advantageously use LNA (Locked Nucleic Acids) primers and probes.

LNA (Locked Nucleic Acids), are nucleic acids analogues containing one or more LNA nucleosides whose major distinguishing characteristic is the presence of a methylene bridge that connects the 2'-oxygen of ribose with the 4'-carbon of the ribose ring. This bridge results in a locked 3'-endo conformation, reducing the conformational flexibility of the ribose and increasing the local organization of the phosphate backbone. These molecular differences between conventional and LNA nucleosides allow increased stability of the nucleic acid duplexes formed between LNAs and other nucleic acids (Braasch & Corey 2001).

The primers and probes of the invention can advantageously be labelled, by any suitable means known in itself by those of skill in the art.

Examples of suitable labels include radioactive labels, fluorescent labels and quenchers, enzymatic labels or labelling with haptens such as biotin, digoxygenin.

Non-limitative examples of suitable fluorescent labels include 6-carboxylfluorescein (FAM), tetrachloro-6-carboxyfluorescein (TET), 6-carboxy-X-rhodamine (ROX). Non-limitative examples of suitable quenchers for labelling dual-labelled probes include 6-carboxy-tetramethyl-rhodamine (TAMRA), DABCYL, Non-Fluorescent Quenchers such as quenchers of the Black Hole Quencher family (BHQ), or including a minor groove binder group (MGB).

The process of the invention can be carried out using any method suitable for PCR amplification of target sequences, including in particular PCR-based DNA arrays.

Particularly preferred methods are however those involving real time PCR amplification as described by Ian M. Mackay in "Real-time PCR in Microbiology : from diagnosis to characterization" (2007) Caister Academic Press, Norfolk, UK.

Real time PCR, also called quantitative real time polymerase chain reaction (qPCR) or kinetic polymerase chain reaction, is used to amplify and simultaneously quantify a targeted DNA molecule. It enables both detection and quantification (as absolute number of copies or relative amount when normalized to DNA input or additional normalizing genes) of a specific sequence in a DNA sample. The procedure follows the general principle of polymerase chain reaction; its key feature is that the amplified DNA is quantified as it accumulates in the reaction in real time after each amplification cycle (Mackay 2007). Two common methods of quantification are the use of fluorescent dyes that intercalate with double-strand DNA, and modified DNA oligonucleotide probes that fluoresce when hybridized with a complementary DNA (Mackay 2007). In the present invention the inventors have shown the second of these two methods, but the other method of quantifying PCR products based upon intercalating fluorescent dyes is also within the scope of the present invention.

The process of the invention can be carried out by performing consecutive PCR assays, for instance a first assay for detecting the presence of the NTNH gene, and a second assay for detecting the presence of any of the BoNT genes (or conversely a first assay for detecting the presence of anyone of the BoNT genes, and a second assay for detecting the presence of the NTNH gene), eventually followed by assays for detecting specifically the BoNT/A, BoNT/B, BoNT/E, and BoNT/F genes.

However, in many cases it will be preferred to carry out simultaneous PCR assays, using one or more multiplex amplification reaction(s). For instance, one can perform a first multiplex amplification reaction to detect simultaneously the NTNH gene and any of the BoNT genes, and a second multiplex amplification reaction to detect specifically the BoNT/A, BoNTB, BoNT/E, and BoNT/F genes. Alternatively one can perform a single PCR multiplex reaction using a PCR-based DNA array to simultaneously detect the presence of the NTNH and a BoNT gene, whilst simultaneously detecting which BoNT gene(s) is present.

For a better understanding of the invention and to show how the same may be carried into effect, there will now be shown by way of example only, specific embodiments, methods and processes according to the present invention with reference to the accompanying drawings in which:
Figure 1 shows the sensitivity of the real-time PCR assays using dilutions of *C. botulinum* broth cultures as template.
Figure 2 shows the detection of *C*. *botulinum* type A in naturally contaminated Foie gras tested in duplicate with BoNT real-time PCR, BoNT/A real-time PCR and NTNH real-time PCR assays.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### EXAMPLE 1 - MATERIALS AND METHODS

### Bacterial strains

The bacterial strains used in this study are listed in Table 1 below. Clostridium strains were maintained on cooked meat medium (CMM) (Oxoid, Basingstoke, Hampshire, England) and stored at 5°C. Spores of Bacillus strains were produced as previously described (Choma et al.2000; Claus & Berkeley. 1986.) and kept in a 30% (vol/vol) glycerol solution at -20°C.

### Cultures

Strains of Clostridium spp. were from the collection of the National Reference Center for Anaerobic bacteria and botulism, Institut Pasteur de Paris, France). They were anaerobically grown overnight at 30°C by transfer of 100 µl CMM cultures to 10 ml of TYG broth. Strains were then subcultured for 9 h at 30°C by transferring 100 µl of the cultures to 10 ml of TYG broth. Cultures of Bacillus spp. strains were grown in J agar (J broth containing tryptone 5 g (Biokar), yeast extract 15 g (Biokar), K₂HPO₄ 3 g, glucose 2 g, water 1 litre, pH 7.4, with agar 15 g (Biokar)) (Choma et al.2000). They were grown by transfer of a single 24 - 48 h colony into 10 ml of J broth.

### Procedure for testing the specimen of "Foie gras"

Samples of 25 g or 50 g of "foie gras" were diluted 10-fold (wt/vol) in prereduced TYG broth containing 200 mg l⁻¹ of D-cycloserine, as described by Sebald and Petit (1997) and under a gas flow of N₂/H₂ (95: 5, vol/vol). After 48 h incubation at 37°C, a 40 ml aliquot of the enrichment broth was collected and kept frozen at -20°C for further mouse bioassay, and a 2 ml aliquot of the same enrichment broth was collected, centrifuged at 9,000 x g for 5 min, and the supernatant discarded. The cell pellet was mixed with the 1 ml of the EasyMag lysis buffer (Biomérieux, Marcy l'étoile, France) and store at 4°C until DNA extraction. DNA extraction was performed with the Nuclisens EasyMag bio-robot (Biomérieux) and following the instructions of the manufacturer. Extracted DNA was stored at -20°C until 5 µl were tested in the PCR reactions.

### Primers and probes

Primers and probes were designed by alignment of the BoNT gene sequences from *C*. *botulinum*, *Clostridium baratii* and *Clostridium butyricum* using the multalin program ClustalW (http://align.genome.jp/). The most highly homologous regions of the sequences allowed a set of universal primers and one probe to be designed for simultaneous detection of the BoNT/A, BoNT/B, BoNT/E, BoNT/F and BoNT/G genes in a real-time PCR (Table 2).

The design of these primers proved to be extremely difficult, in particular due to the low levels of homology between the various sequences of the BoNT genes from multiple strains of serotypes A, B, E and F. Strains for the same serotype also showed significant sequence variation within each serotype.

**Table 2 Primers and probes.**

| Oligonucleotide ^{a} | DNA sequences (5' → 3') |
|---|---|
| A1 (SEQ ID NO:1) | GGAGTCACTTGAAGTTGATACAAATC |
| A2 (SEQ ID NO:2) | GCTAATGTTACTGCTGGATCTGTAG |
| A3 (SEQ ID NO:3) | FAM-TCTTTTAGGTGCAGGCAAATTT-BHQ |
| B1 (SEQ ID NO:4) | GATGAACAGCCAACATATAGTTGTCA |
| B2 (SEQ ID NO:5) | GTTTCCTTTTTACCTCTTTTAAGTACCATT |
| B3 (SEQ ID NO:6) | FAM-TGATGAKATAGGATTGATTGGTATTCA-BHQ |
| E1 (SEQ ID NO:7) | CTATCCAAAATGATGCTTATATACCAAA |
| E2 (SEQ ID NO:8) | GGCACTTTCTGTGCATCTAAATA |
| E3 (SEQ ID NO:9) | FAM-ATGATTCTAATGGAACAAGTGATATAGAACAACATGATGT-BHQ |
| F1 (SEQ ID NO:10) | GCAATATAGGATTACTAGGTTTTCATTC |
| F2 (SEQ ID NO:11) | GAAATAAAACTCCAAAAGCATCCATT |
| F3 (SEQ ID NO:12) | FAM-TTGGTTGCTAGTAGTTGGTATTATAACAA-BHQ |
| U1 (SEQ ID NO:13) | AATAATTCDGG**M**TGGAAA**R**TATC |
| U2 (SEQ ID NO:14) | CCATTDAT**R**TAAA**KTY**TAG |
| U3 (SEQ ID NO:15) | FAM-ATYATTAGTDATAGTTACAAAAA**W**CCA-BHQ |
| NT1(SEQ ID NO:16) | A**R**TGG**W**ATGGGAAC**H**ATG |
| NT2(SEQ ID NO:17) | GC**W**GGATCAA**Y**ATA**R**AATT |
| NT3(SEQ ID NO:18) | FAM-CAACCATTT**Y**TAAC**MY**ATAAATA-MGB |

| | |
|---|---|
| ^{a} in the sequence of oligonucleotides W is (A,T); R is (A,G); K is (G,T); Y is (C,T); H is (A,T,C); D is (G,A,T) and M is (A,C). Underlined letters represent LNA bases. FAM = 6-carboxylfluorescein BHQ = Black Hole Quencher MGB = Minor Groove Binder | |

Four primer pairs and 4 probes with each being specific for BoNT/A, BoNT/B, BoNT/E, or BoNT/F were designed (Table 2). They were selected from the non-homologous regions of the BoNT/A, BoNTB, BoNT/E, and BoNT/F genes to enable a toxin type-specific identification. Finally, one primer pair and one probe were designed in the most highly homologous regions of the NTNH gene sequences (Table 2). In order to permit mismatches within some primers or probes site, the inventors used Locked Nucleic Acid (LNA) bases which bracket the bases in some oligonucleotide sequences that showed degeneracy among strains in order to increase specificity for the conserved nucleotides. All probes were 5'-labeled with 6-carboxylfluorescein (FAM) and 3'-labeled with Black Hole Quencher (BHQ), except for the NTNH probe which was 3'-labeled with a minor groove binder ligand (MGB).

In order to develop assays targeting the BoNT and the NTNH genes a data bank collecting all available DNA sequences encoding for bontA, bontB, bontE and bontF and ntnh has been achieved. It includes the sequences of 4 BoNT/A variants (A1, A2, A3, A4), 7 BoNT/B variants (proteolytic group: B1, B2, B3, Ba, Bf, Ab ; non proteolytic group : B), 4 BoNT/E variants (non proteolytic group : E1, E2, E3, E6 and 2 variants for *C*. *butyricum* :E4, E5), 4 BoNT/F variants (including the variant from *C*. *baratii*), and NTNH gene sequences, including sequences published in GenBank and sequences obtained by the inventors. Alignment of the BoNT gene sequences and identification of the most conserved regions within the BoNT genes allowed a set of primers and one single probe to be designed for screening any *C*. *botulinum* types A, B, AB, E or F and the toxigenic non-botulinum Clostridium spp., *C*. *butyricum* type E and *C*. *baratii* type F. The real-time PCR assay based on the highly homologous regions of the NTNH gene tested positive all these strains and additionally the strain expressing toxin G. None of the *C*. *sporogenes* or C. botulinum-like strains or other bacterial species yielded a positive signal by these two PCR assays.

### PCR conditions

BoNT type-specific amplifications were performed in a total volume of 25 µl of 1X qPCR master mix buffer from Eurogentec (Liege, Belgium), containing, 0.3 µM of each primer, 0.1µM of probe and 5µl of template DNA. Amplifications were carried out on an ABI Prism 7700 Sequence Detection System (Applied Biosystems, Courtaboeuf, France) according to the following temperature profile: one cycle of 95°C for 10 min and 35 cycles of 15 s at 95°C and 1 min at 60°C.

Universal PCR tests targeting the most highly homologous regions of either the BoNT or NTNH genes were performed in a total volume of 25 µl of 1X concentration of a LightCycler-Faststart DNA master hybridization probes mix (Roche Diagnostics, Meylan, France), containing, 4.2 mM MgCl₂, 0.9 µM of each primer, 0.4µM of probe and 5µl of template DNA. Amplifications were carried out on the ABI Prism 7700 Sequence Detection System (Applied Biosystems) according to the following temperature profile: one cycle of 95°C for 10 min, 5 cycles of 95°C for 15 s, 45°C for 20 s with 2.5 minutes transition time between 45°C and 95°C, and 35 cycles of 15 s at 95°C and 20 s at 50°C with 2.5 minutes transition time between 50°C and 95°C.

Positive controls and two negative controls containing all reagents except DNA template were included with every amplification set. To avoid contamination, sample preparation, PCR amplification and PCR detection were performed in different rooms.

### Sensitivity of the real-time PCR

The limit of detection (LOD) of the PCR test based on universal BoNT primers and probe was determined with either total genomic DNA or recombinant plasmids. Genomic DNA from *C*. *botulinum* types A (strain Hall A), B (strain IPBL6), E (strain HV) and F (strain NCIMB 10658) was extracted and purified as previously described by Popoff et al. (1985) and quantified by spectrophotometry. The recombinant plasmids which contained amplified fragments from bont/A, bont/B, bont/E and bont/F genes were those described by Fach et al. (2002). Plasmid DNA concentrations were determined by fluorimeter and the copy number of each plasmid was calculated according to its size.

The LOD of the four toxin type-specific PCR tests, each being specific for either BoNT/A, BoNT/B, BoNT/E, or BoNT/F was determined with either serial dilutions of total purified genomic DNA or with serial dilutions of *C*. *botulinum* broth cultures which were counted microscopically in a Petrov chamber before DNA extraction with the InstaGene™ Matrix (Bio-Rad Laboratories, Marnes-La-Coquette, France), as previously described by Fach et al (2002).

The LOD of the universal PCR test targeting the NTNH genes was determined with serial dilutions of *C*. *botulinum* broth cultures which were counted microscopically in a Petrov chamber before DNA extraction with the InstaGene™ Matrix (Bio-Rad), as previously described by Fach et al (2002).

### EXAMPLE 2 - RESULTS

### Specificity

Purified genomic DNA templates from *C*. *botulinum* type A (n = 8), *C*. *botulinum* type B (n = 8), *C*. *botulinum* type AB (n = 2), *C*. *botulinum* type E (n = 4), and *C*. *botulinum* type F (n = 1) yielded expected specific positive signals by the four toxin type-specific PCR tests (Table 1). The assays tested negative strains expressing toxin types C, D, and G. It should be noted that type G strains are extremely rare and only one of two reported strains was incorporated in the panel. The toxigenic non-botulinum Clostridium spp., *C*. *butyricum* type E (n = 2) and *C*. *baratii* type F (n = 1) were tested positive for the presence of BoNT/E and BoNT/F genes respectively. No amplification was observed when non-botulinum toxin expressing Clostridium spp. (21 strains) or other bacteria (3 strains) DNA was used as template. To confirm that botulinum toxin was expressed by the toxic clostridial strains used in this study, mouse neutralization bioassay of culture supernatants was performed at the Pasteur Institute (data not shown).

**Table 1. Results of PCR-assays on pure cultures of Clostridium botulinum and other species**

| Species | Toxin type | Number of strains tested | NTNH PCR | BoNT PCR | BoNT/A PCR | BoNT/B PCR | BoNT/E PCR | BoNT/F PCR |
|---|---|---|---|---|---|---|---|---|
| *Clostridium botulinum* type A | A | 8 | + | + | + | - | - | - |
| *C. botulinum* type B | B | 8 | + | + | - | + | - | - |
| *C. botulinum* type AB | AB | 2 | + | + | + | + | - | - |
| *C. botulinum* type E | E | 4 | + | + | - | - | + | - |
| *C. butyricum* type E | E | 2 | + | + | - | - | + | - |
| *C. botulinum* type F | F | 1 | + | + | - | - | - | + |
| *C. baratii* type F | F | 1 | + | + | - | - | - | + |
| *C. botulinum* type C | C | 1 | nt^{a} | - | - | - | - | - |
| *C. botulinum* type D | D | 1 | nt | - | - | - | - | - |
| *C. botulinum* type G | G | 1 | + | - | - | - | - | - |
| *C. tetani* | NT^{b} | 1 | - | - | - | - | - | - |
| *C. butyricum* | NT | 2 | - | - | - | - | - | - |
| *C. baratii* | NT | 1 | - | - | - | - | - | - |
| *C. sporogenes* | NT | 1 | - | - | - | - | - | - |
| *C. spirogenes* | NT | 1 | - | - | - | - | - | - |
| *C. subterminale* | NT | 1 | - | - | - | - | - | - |
| *C. sordellii* | NT | 1 | - | - | - | - | - | - |
| *C. septicum* | NT | 1 | - | - | - | - | - | - |
| *C. oedematiens* | NT | 1 | - | - | - | - | - | - |
| *C. mangenotti* | NT | 1 | - | - | - | - | - | - |
| *C. chauvoei* | NT | 1 | - | - | - | - | - | - |
| *C. bifermentans* | NT | 1 | - | - | - | - | - | - |
| *C. beijerinckii* | NT | 1 | - | - | - | - | - | - |
| *C. difficile* | NT | 2 | - | - | - | - | - | - |
| *C. perfringens* | NT | 5 | - | - | - | - | - | - |
| *Bacillus cereus* | NT | 1 | - | - | - | - | - | - |
| *B. thuringiensis* | NT | 1 | - | - | - | - | - | - |
| *Streptococcus faecalis* | NT | 1 | - | - | - | - | - | - |

| | | | | | | | | |
|---|---|---|---|---|---|---|---|---|
| ^{a}nt: Not tested; ^{b}NT: Non BoNT producing strains | | | | | | | | |

The universal PCR tests targeting the most highly homologous regions of either the BoNT or NTNH genes yielded the expected positive signals for *C*. *botulinum* types A, B, AB, E or F and for the toxigenic non-botulinum Clostridium spp., *C*. *butyricum* type E and *C*. *baratii* type F. Additionally, the NTNH PCR assay tested a positive strain expressing toxin G. None of the *C*. *sporogenes* or C. botulinum-like strains or other bacterial species yielded a positive signal by these two PCR assays.

### Sensitivity

Purified genomic DNA from *C*. *botulinum* types A (strain Hall A), B (strain IPBL6), E (strain HV) and F (strain NCIMB 10658) was quantified by spectrophotometry and converted to genomic copy number by assuming that the size of each strain's genome is equal to that of the sequenced *C*. *botulinum* type A Hall strain (3.9 Mbp) (http://www.sanger.ac.uk/Projects/C_botulinum/). Using purified genomic DNA as template, limits of detection (LOD) was determined by testing serial 10-fold dilutions of DNA in duplicate. The LOD for each toxin type ranged between 250 and 2500 genome copies by using the universal BoNT PCR test (Table 3). These data correlate with the LOD obtained with the recombinant plasmids which was 500 copies for BoNT/A, 250 copies for BoNT/B, 350 copies for BoNT/E and 450 copies for BoNT/F (data not shown). The limit of detection as determined with genomic DNA from *C*. *botulinum* strains was 10-fold improved and ranged between 25 and 250 genome copies when using the four toxin type-specific PCR tests. The LOD was lowest for type A strain Hall (25 genome copies) and highest for type E strain HV (250 genome copies), (Table 3).

**Table 3. Sensitivity of the real-time PCR assays using dilutions of purified genomic DNA as template.**

| Strain | Toxin type | Toxin type-specific PCR | | Universal BoNT PCR | |
|---|---|---|---|---|---|
| | | Purified genomic DNA | | Purified genomic DNA | |
| | | fg DNA | Genome equivalents ^{a} | fg DNA | Genome equivalents ^{a} |
| *C*. *botulinum C*. *botulinum* A (strain Hall ) | a | 100 | 25 | 1000 | 250 |
| *C*. *botulinum* B (strain BL6) | b | 100-1000 | 25-250 | 1000 | 250 |
| *C*. *botulinum* E (strain HV) | e | 1000 | 250 | 10000 | 2500 |
| *C*. *botulinum* F (strain NCIMB 10658) | f | 100-1000 | 25-250 | 10000 | 2500 |

| | | | | | |
|---|---|---|---|---|---|
| ^{a} Genome equivalents are calculated based on the mass of the *C*. *botulinum* type A Hall strain as described in the text. | | | | | |

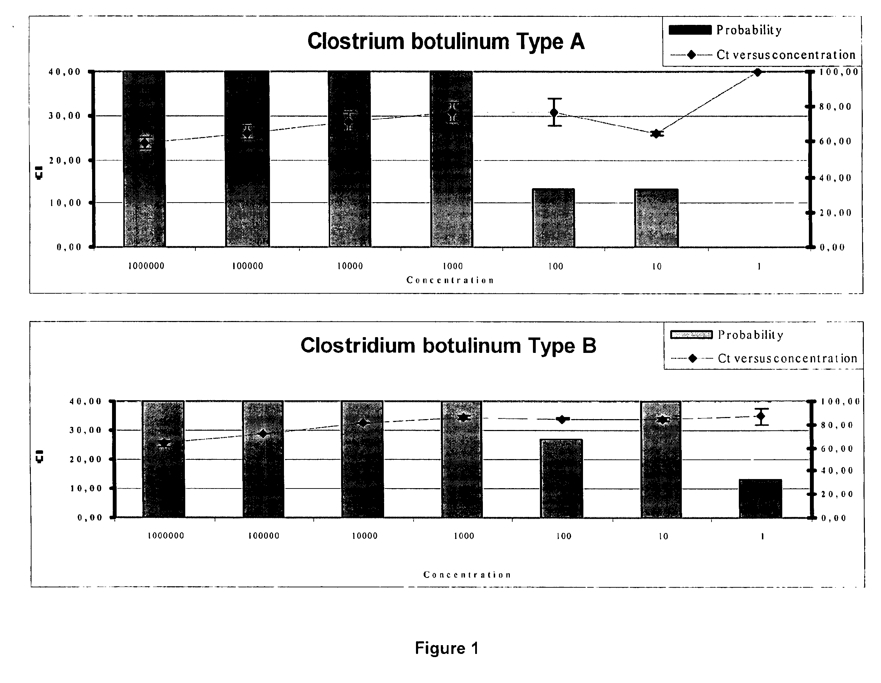

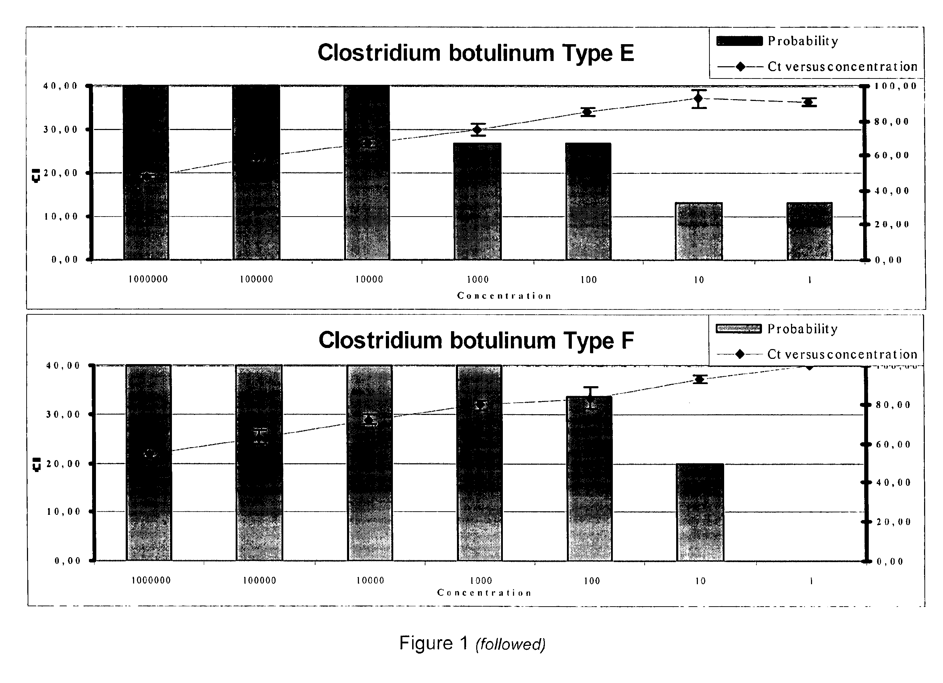

Limits of detection of the four toxin type-specific PCR tests was further determined with triplicates of serial dilutions of *C*. *botulinum* broth cultures which were counted microscopically in a Petrov chamber before DNA extraction. Each DNA triplicate was tested in double in the PCR tests so that six Ct values were obtained for each bacterial dilution. This allowed to the inventors to determine mean Ct values for each bacterial dilution, the LOD and the probability of detection of the methods (Figure 1). Concentrations of 10³ cells ml⁻¹ of *C*. *botulinum* type A, B and F (approximately 25 copies in the PCR tube) and higher concentrations yielded positive results and 100% probability of detection. Usually, the mean Ct values were around 30 for the concentration of 10³ cells ml⁻¹. For concentrations under 10³ cells ml⁻¹ (less than 25 copies in the PCR tube) the mean Ct values were higher than 30 and the probability of detection was lower than 100%. For *C*. *botulinum* type E, 10⁴ cells ml⁻¹ and higher concentrations tested positive with 100% probability of detection (Figure 1).

Limits of detection (LOD) of the NTNH PCR test was determined using serial 10-fold dilutions of *C*. *botulinum* broth cultures as template. Each DNA extract was tested in duplicate. Limits of detection for each toxin type ranged between 3 and 25 genome copies in the PCR tube. The limit of detection was lowest (3 genome copies) for strains of *C*. *botulinum* type A, B and E and highest for *C*. *botulinum* type F (25 genome copies). Taken together, our results indicate that the sensitivity level of the NTNH PCR allows the detection of few ntnh gene copies (less than 25 gene copies) in the PCR tube (data not shown).

### Detection of C. botulinum in a specimen of "Foie gras"

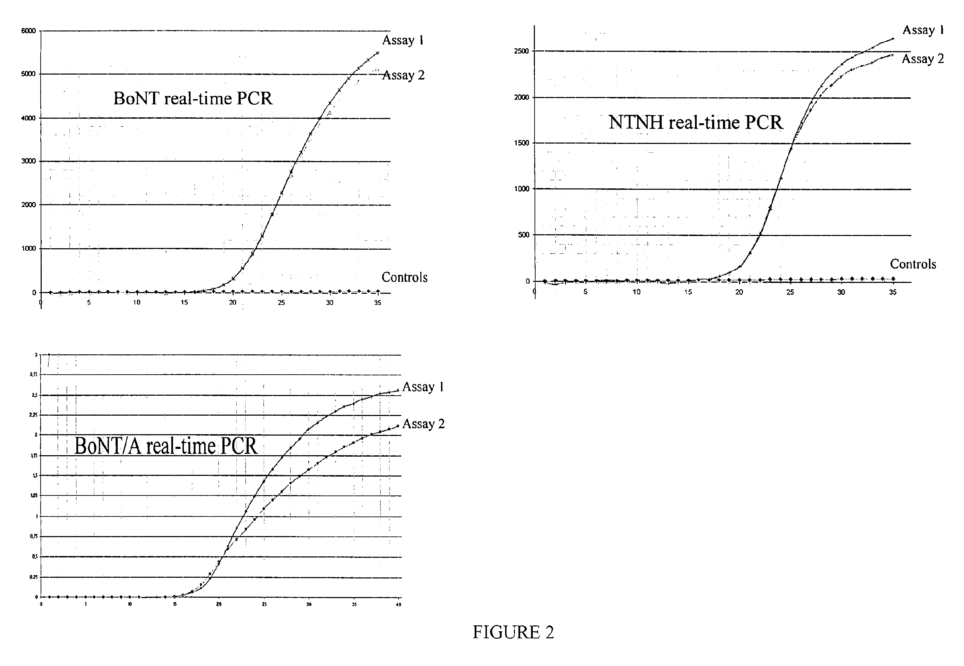

In order to test the applicability of the PCR assays in investigation of food the inventors have tested one specimen of "foie gras" which had been collected during a food poisoning outbreak occurring in France in December 2007. After a 48h enrichment in anaerobic conditions and DNA extraction using the Nuclisens EasyMag bio-robot, the sample tested positive by universal PCR tests targeting the most highly homologous regions of either the BoNT or NTNH genes. In addition, it yielded also a positive signal with BoNT/A type-specific PCR test (Figure 2). The specimen has been confirmed positive for *C*. *botulinum* type A with the reference lethality test on mice when tested at Pasteur Institute (Reference Center for anaerobic bacteria, Paris, France).

### REFERENCES

- Braasch, D. A. & Corey, D. R. (2001). Locked nucleic acid (LNA): fine-tuning the recognition of DNA and RNA. Chemistry & biology 55, 1-7.
- Choma, C., Guinebretiere, M.H., Carlin, F., Schmitt, P., Velge, P., Granum, P.E. and Nguyen-the, C. (2000). Prevalence, characterisation and growth of Bacillus cereus in commercial cooked chilled foods containing vegetables. J Appl Microbiol 88, 617-625.
- Claus, D. and Berkeley, R.C.W. (1986) Genus Bacillus Cohn 1872, 174AL, p. 1105-1139. In P. H. A. Sneath, N. S. Mair, M. E. Sharpe, and J. G. Holt (ed.), Bergey's Manual of Systematic Bacteriology, vol. 2. Williams & Wilkins, Baltimore.
- East, A.K. and Collins, M.D. (1994) Conserved structure of genes encoding components of botulinum neurotoxin complex M and the sequence of the gene coding for the nontoxic component in nonproteolytic Clostridium botulinum type F, Curr. Microbiol. 29, 69-77.
- Fach, P., Perelle, S., Dilasser, F., Grout, J., Dargaignaratz, C., Botella, L., Gourreau, J.M., Carlin, F., Popoff, M.R. and Broussolle, V. (2002) Detection by PCR-enzyme-linked immunosorbent assay of Clostridium botulinum in fish and environmental samples from a coastal area in northern France. Appl Environ Microbiol 68, 5870-5876.
- Gimenez, D.F. and Gimenez, J.A. (1993) Serological subtypes of botulinal toxins. In: DasGupta BR. , editor. Botulinum and tetanus neurotoxins. New York: Plenum Press;. pp. 421-431.
- Hauser, D., Gibert, M., Marvaud, J.C., Eklund M.W. and Popoff, M.R. (1995) Botulinal neurotoxin C1 complex genes, clostridial neurotoxin homology, and genetic transfer in Clostridium botulinum. Toxicon 33, 515-526.
- Herreros, J., Lalli, G., Montecucco, C. and Schiavo, G. (1999). Pathophysiological properties of clostridial neurotoxins, p. 202-228. In J. E. Alouf and J. H. Freer (ed.), The comprehensive sourcebook of bacterial protein toxins, vol. 2. Academic Press, London.
- Hill, K.K., Smith, T.J., Helma, C.H., Ticknor, L.O., Foley, B.T., Svensson, R.T., Brown, J.L., Johnson, E.A., Smith, L.A., Okinaka, R.T., Jackson, P.J. and Marks, J.D. (2007) Genetic diversity among Botulinum Neurotoxin-producing clostridial strains. J Bacteriol 189, 818-832.
- Mackay, I.M., (2007) Real-time PCR in Microbiology, from diagnosis to characterization. Caister Academic Press, Norfolk, UK.
- Maksymowych, A.B., Reinhard, M., Malizio, C.J., Goodnough, M.C., Johnson EA, and Simpson L.L. (1999) Pure botulinum neurotoxin is absorbed from the stomach and small intestine and produces peripheral neuromuscular blockade. Infect Immun 67, 4708-4712.
- McCroskey, L.M., Hatheway, C.L., Fenicia, L., Pasolini, B. and Aureli, P. (1986) Characterization of an organism that produces type E botulinal toxin but which resembles Clostridium butyricum from the faeces of an infant with type E botulism, J Clin Microbiol. 23, 201-202.
- McCroskey, L.M., Hatheway, C.L., Woodruff, B.A., Greenberg, J.A. and Jurgenson, P. (1991) Type F botulism due to neurotoxigenic Clostridium baratii from an unknown source in an adult, J Clin Microbiol 29, 2618-2620.
- Popoff, M.R., Guillou, J.P. and Carlier J.P. (1985) Taxonomic position of lecithinase negative strains of Clostridium sordellii. J Gen Microbiol 131, 1697-1703.
- Popoff, M.R. (1995). Ecology of neurotoxigenic strains of Clostridia, p. 1-29. In C. Montecucco (ed.), Clostridial neurotoxins, vol. 195. Springer-Verlag, Hiedelberg.
- Raphael, B.H. and Andreadis, J.D. (2007) Real-time PCR detection of the nontoxic nonhemagglutinin gene as a rapid screening method for bacterial isolates harbouring the botulinum neurotoxin (A-G) gene complex. J Microbiol Methods 71, 343-346.
- Rodriguez Jovita, M., Collins, M.D. and East A.K. (1998) Gene organization and sequence determination of the two botulinum neurotoxin gene clusters in Clostridium botulinum type A(B) strain NCTC 2916. Curr Microbiol 36, 226-231.
- Schiavo, G., Matteoli, M. and Montecucco, C. (2000). Neurotoxins affecting neuroexocytosis. Physiol Rev 80, 717-766.
- Sebald, M. and Petit, J.C. (1997). Méthodes de laboratoire. Bactéries anaérobies et leur identification. 2nd edn. Institut pasteur, Paris.

## Claims

1. A process for the detection of neurotoxin producing clostridia in a sample, wherein said process comprises contacting said sample or DNA isolated therefrom with a pair of primers derived from the NTNH gene, said pair of primers comprising:
- a primer defined by the sequence SEQ ID NO: 16, or a fragment of at least 15 nucleotides thereof;
- a primer defined by the sequence SEQ ID NO: 17, or a fragment of at least 15 nucleotides thereof;
and detecting the presence or the absence of an amplification product.

2. The process according to claim 1, wherein said amplification products is detected using a probe consisting of SEQ ID NO: 18 or a fragment of at least 15 nucleotides thereof.

3. The process according to any one of claims 1 or 2, further comprising contacting said sample or DNA isolated therefrom with a pair of primers derived from the BoNT gene, said pair of primers comprising:
- a primer defined by the sequence SEQ ID NO: 13, or a fragment of at least 15 nucleotides thereof;
- a primer defined by the sequence SEQ ID NO: 14, or a fragment of at least 15 nucleotides thereof.
and detecting the presence or the absence of an amplification product.

4. The process according to claim 3, wherein said amplification products are detected using a probe consisting of: SEQ ID NO: 15 or a fragment of at least 15 nucleotides thereof.

5. The process according to any one of claims 1 to 4, further comprising contacting said sample or DNA isolated therefrom with one or more pair(s) of primers selected among:
a) a pair of primers derived from the BoNT/A gene, said pair of primers comprising:
- a primer defined by the sequence SEQ ID NO: 1, or a fragment of at least 15 nucleotides thereof;
- a primer defined by the sequence SEQ ID NO: 2, or a fragment of at least 15 nucleotides thereof;
b) a pair of primers derived from the BoNT/B gene, said pair of primers comprising:
- a primer defined by the sequence SEQ ID NO: 4, or a fragment of at least 15 nucleotides thereof;
- a primer defined by the sequence SEQ ID NO: 5, or a fragment of at least 15 nucleotides thereof;
c) a pair of primers derived from the BoNT/E gene, said pair of primers comprising:
- a primer defined by the sequence SEQ ID NO: 7, or a fragment of at least 15 nucleotides thereof;
- a primer defined by the sequence SEQ ID NO: 8, or a fragment of at least 15 nucleotides thereof;
d) a pair of primers derived from the BoNT/F gene, said pair of primers comprising:
- a primer defined by the sequence SEQ ID NO: 10, or a fragment of at least 15 nucleotides thereof;
- a primer defined by the sequence SEQ ID NO: 11, or a fragment of at least 15 nucleotides thereof;
and detecting the presence or the absence of an amplification product for each pair of primer.

6. The process according to claim 5, wherein said amplification product is detected using a probe specific for one BoNT serotype selected from the group consisting of: SEQ ID NO: 3 for BoNT/A; SEQ ID NO: 6 for BoNT/B; SEQ ID NO: 9 for BoNT/E; SEQ ID NO: 12 for BoNT/F; or a fragment of at least 15 nucleotides of any one thereof.

7. The process according to any one of claims 2 to 6, wherein said probes are labelled with at least one fluorescent label.

8. The process according to any one of claims 3 to 7, wherein said process comprises a multiplex amplification reaction.

9. The process according to any one of claims 3 to 7, wherein said process comprises a series of independent amplification reactions.

10. The process according to any one of claims 2 to 9, wherein said amplification reaction is a real time PCR reaction.

11. A kit for the detection of botulinum neurotoxin producing organisms, comprising a pair of primers as defined in claim 1, and optionally a probe as defined in claim 2.

12. The kit according to claim 11, further comprising a pair of primers as defined in claim 3, and optionally a probe as defined in claim 4.

13. The kit according to any of claims 11 or 12, further comprising one or more pair(s) of primers as defined in claim 5, and optionally one or more probe(s) as defined in claim 6.

14. An isolated nucleic acid molecule consisting of the amplification product resulting from a process according to any one of claims 1 to 10.


SEQUENCE LISTING

<110>  AGENCE FRANCAISE DE SECURITE SANITAIRE DES ALIMENTS

<120>  PROCESS FOR DETECTING BOTULINUM NEUROTOXIN PRODUCING ORGANISMS

<130>  MJP/DT/mad-F1159/5-EP

<160>  18

<170>  PatentIn version 3.3

<210>  1
<211>  26
<212>  DNA
<213>  Artificial

<220>
<223>  BoNT/A Primer 1

<400>  1
ggagtcactt gaagttgata caaatc                                          26

<210>  2
<211>  25
<212>  DNA
<213>  Artificial

<220>
<223>  BoNT/A primer 2

<400>  2
gctaatgtta ctgctggatc tgtag                                           25

<210>  3
<211>  22
<212>  DNA
<213>  Artificial

<220>
<223>  BoNT/A probe 3

<400>  3
tcttttaggt gcaggcaaat tt                                              22

<210>  4
<211>  26
<212>  DNA
<213>  Artificial

<220>
<223>  BoNT/B primer 1

<400>  4
gatgaacagc caacatatag ttgtca                                          26

<210>  5

<211>  30
<212>  DNA
<213>  Artificial

<220>
<223>  BoNT/B primer 2

<400>  5
gtttcctttt tacctctttt aagtaccatt                                        30


<210>  6
<211>  27
<212>  DNA
<213>  Artificial

<220>
<223>  BoNT/B probe 1

<400>  6
tgatgakata ggattgattg gtattca                                           27


<210>  7
<211>  28
<212>  DNA
<213>  Artificial

<220>
<223>  BoNT/E primer 1

<400>  7
ctatccaaaa tgatgcttat ataccaaa                                          28


<210>  8
<211>  23
<212>  DNA
<213>  Artificial

<220>
<223>  BoNT/E primer 2

<400>  8
ggcactttct gtgcatctaa ata                                               23


<210>  9
<211>  40
<212>  DNA
<213>  Artificial

<220>
<223>  BoNT/E probe 1

<400>  9
atgattctaa tggaacaagt gatatagaac aacatgatgt                             40


<210>  10
<211>  28

<212>  DNA
<213>  Artificial

<220>
<223>  BoNT/F primer 1

<400>  10
gcaatatagg attactaggt tttcattc                                          28


<210>  11
<211>  26
<212>  DNA
<213>  Artificial

<220>
<223>  BoNT/F primer 2

<400>  11
gaaataaaac tccaaaagca tccatt                                            26


<210>  12
<211>  29
<212>  DNA
<213>  Artificial

<220>
<223>  BoNT/F probe 1

<400>  12
ttggttgcta gtagttggta ttataacaa                                         29


<210>  13
<211>  23
<212>  DNA
<213>  Artificial

<220>
<223>  BoNT/Universal Primer 1

<400>  13
aataattcdg gmtggaaart atc                                               23


<210>  14
<211>  19
<212>  DNA
<213>  Artificial

<220>
<223>  BoNT/Universal Primer 2

<400>  14
ccattdatrt aaaktytag                                                    19


<210>  15
<211>  27
<212>  DNA

<213>  Artificial

<220>
<223>  BoNT/Universal probe 1

<400>  15
atyattagtd atagttacaa aaawcca                                          27


<210>  16
<211>  18
<212>  DNA
<213>  Artificial

<220>
<223>  NTNH primer 1

<400>  16
artggwatgg gaachatg                                                    18


<210>  17
<211>  19
<212>  DNA
<213>  Artificial

<220>
<223>  NTNH primer 2

<400>  17
gcwggatcaa yataraatt                                                   19


<210>  18
<211>  23
<212>  DNA
<213>  Artificial

<220>
<223>  NTNH probe 1

<400>  18
caaccattty taacmyataa ata                                              23